# EUROPÄISCHE PATENTSCHRIFT

(11) **EP 2 578 514 B1**
(45) Veröffentlichungstag und Bekanntmachung des Hinweises auf die Patenterteilung: **29.07.2020**
(21) Anmeldenummer: 12006379.7
(22) Anmeldetag: 11.09.2012
(51) Int. Cl.: B65D 55/02, A61B 10/00

(54) **Behälter mit Verschlusssicherung**
Container with safety lock
Récipient doté d'une sécurisation de fermeture

(30) Priorität: 03.10.2011 CH 16162011
(43) Veröffentlichungstag der Anmeldung: 10.04.2013
(73) Patentinhaber: Berlinger & Co., 9608 Ganterschwil (CH)
(72) Erfinder: Oertli, Christian, 9621 Oberhelfenschwil (CH)
(74) Vertreter: Quehl, Horst Max

(56) Entgegenhaltungen:
- EP-A2- 0 381 516
- DE-U1- 29 515 681
- US-A- 2 958 439
- US-A- 5 690 246

## Beschreibung

Die Erfindung betrifft einen Behälter mit den Merkmalen des Oberbegriffs des Patentanspruchs 1.

Verschlusssicherungen für Behälter werden dazu benötigt, einen unbefugten und nicht nachweisbaren Zugang zu dem Behälterinhalt unmöglich zu machen, wie es z.B. für die Aufbewahrung einer Urinprobe einer Dopingkontrolle erforderlich ist. Ein Beispiel einer an einem Probenbehälter vorgesehenen Verschlussicherung ist durch die EP10405191 bekannt. Die EP0381516A2, die als nächstliegender Stand der Technik betrachtet wird, offenbart einen Behälter gemäss dem Oberbegriff des Anspruchs 1.

Der Erfindung liegt die Aufgabe zugrunde, einen Behälter mit Verschlusssicherung zu finden, die mit geringem Aufwand durch einen Benutzer an einem Behälter bekannter Ausführung montierbar und schliessbar ist und die ebenso wie der Behälter, auf einfache bzw. kostengünstige Weise im Kunststoffspritzverfahren herstellbar ist.

Die Lösung der Aufgabe der Erfindung erfolgt aufgrund der kennzeichnenden Merkmale des Patentanspruchs 1.

Vorteilhafte Ausgestaltungen der Erfindung sind Gegenstand der abhängigen Patentansprüche und der folgenden Beschreibung anhand der Zeichnungen zu entnehmen. Es zeigt:
Fig.1 eine perspektivische Darstellung eines Behälters mit einer an ihm vorgesehene, erfindungsgemässen Verschlusssicherung, bevor diese am Behälter geschlossen wird,
Fig.2 einen Querschnitt durch den Behälter mit geschlossener Verschlusssicherung,
Fig. 3 eine Aufsicht auf den Behälter nach Fig.2,
Fig.4 einen vergrösserten Teilquerschnitt im Bereich der Rastmittel der Verschlusssicherung.

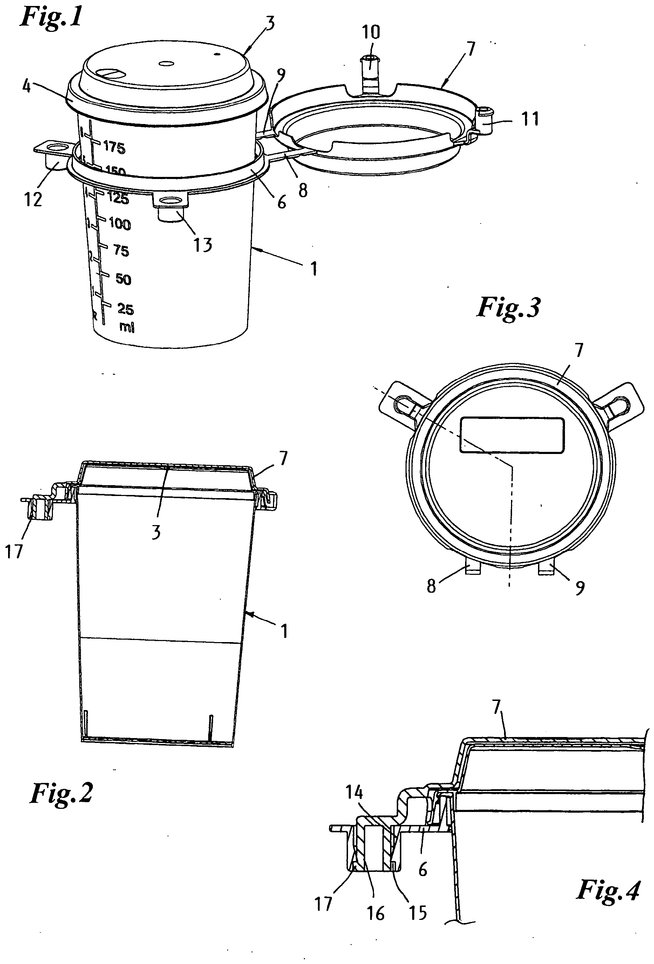

Der Behälter 1 hat beispielsweise die in Fig.1 gezeigte, sich nach unten verjüngende, konische Form eines Bechers mit einem radial nach aussen abstehenden Öffnungsrand, über dem ein Schnappdeckel 3 bekannter Ausführung mit seinem übergreifenden Rand 4 durch einfaches Aufdrücken verrastet ist. Eine zuvor in den Behälter eingefüllte Substanz, wie z.B. die Urinprobe eines Dopingtestes ist folglich luftdicht im Behälter 1 eingeschlossen.

Die für eine fälschungssichere Aufbewahrung des Behälterinhaltes vorgesehene Verschlusssicherung wird als Deckelsicherung anschliessend am gefüllten und verschlossenen Behälter 1 zerstörungsfrei unlösbar befestigt. Hierzu hat die Verschlusssicherung eine von unten auf den Behälter 1 bis in Kontakt mit dem Öffnungsrand aufschiebbaren Käfigring 6 und einen über den Schnappdeckel 3 klappbaren Deckelteil 7, der in dieser, parallel zum Schnappdeckel 3 ausgerichteten Position entsprechend der Darstellung in Fig. 1, mit dem Käfigring 6 zerstörungsfrei unlösbar verrastet ist.

Um die hierfür erforderliche Klappbewegung des Deckelteiles 7 zu ermöglichen, ist dieser gelenkig oder scharnierartig, z.B. über zwei biegsame Stege 8,9 seitlich mit dem Käfigring 6 einstückig verbunden.

Zur zerstörungsfrei unlösbaren Verrastung des Deckelteiles 7 sind auf der diametral gegenüberliegenden Seite dieser biegsamen Verbindung hierzu geeignete Rastmittel vorgesehen. Diese Rastmittel haben an einem dieser miteinander verrastenden Teile 7 ein Rasteingriffsteil 10, 11 und am anderen Teil 6 jeweils eine Rastaufnahme 12, 13, die auf einfache Weise beim Überklappen des Deckelteiles 7 auf den Käfigring miteinander verrastbar sind.

Entsprechend dem dargestellten Ausführungsbeispiel der Erfindung befinden sich am Käfigring 6 zwei radial von ihm abstehende, einen Ringquerschnitt aufweisende Rastaufnahmen 12, 13, an deren Innenseite mittels eines nasenförmigen Querschnittes, bzw. einer sich nach unten konisch verjüngenden Innenfläche 14 eine umlaufende Rastschulter 15 gebildet ist. Zur leichten Ausführbarkeit der Rastschulter 15 im Spritzgiessverfahren sind die Rastaufnahmen 12, 13 beidseitig offen ausgeführt.

Die Rasteingriffsteile 10, 11 bilden jeweils einen aussen am Deckelteil angeformten, in die Rastaufnahmen 12, 13 einfügbaren Hohlzapfen 16, der an einem Teil seines Umfanges ebenfalls schulterförmig einen abstehenden im Querschnitt nasenförmigen Querschnitt aufweist, durch den eine Rastklinke 17 gebildet ist. Dieser Rastklinkenteil 17 erstreckt sich beispielsweise entlang eines Umfanges von etwas mehr als 180° und ist somit bei ausreichender Formstabilität für den sicheren Rasteingriff ausreichend ausbiegbar, um den Rasteingriff herzustellen.

Ein Lösen des beschriebenen Rasteingriffes ist nur durch Zerstörung oder sichtbare Beschädigung an dem Deckelteil 7 möglich, beispielsweise durch Abschneiden der Rastaufnahmen 12, 13 einschliesslich der in ihnen eingeschlossenen Rasteingriffsteile 10, 11.

## Patentansprüche

1. Behälter mit einem nach aussen abstehenden Öffnungsrand, einem mit seinem übergreifenden Rand (4) mit diesem in dichtend verrastetem Eingriff verbundenen Schnappdeckel (3) und mit einer Verschlusssicherung die diesen zerstörungsfrei unlösbar mit dem Behälter (1) verbindet, wobei die Verschlusssicherung einerseits einen Käfigring (6) aufweist, so dass dieser den Behälter (1) unterhalb seines Öffnungsrandes umschliesst und andererseits einen Deckelteil (7), der den Schnappdeckel (3) des Behälters (1) teilweise überdeckt, wobei der Käfigring (6) mit dem Deckelteil (7) scharnierartig verbunden ist und an mindestens einem der Scharnierverbindung (8,9) quer zum Behälter gegenüberliegenden Bereich zerstörungsfrei unlösbar in Eingriff stehende Rastmittel (10 - 13) aufweist, die den Käfigring (6) und den auf diesen übergeklappten Deckelteil (7) der Verschlusssicherung miteinander verrasten, so dass diese den Schnappdeckel (3) in sich einschliessen, **dadurch gekennzeichnet, dass** der Käfigring (6) von unten auf den Behälter (1) bis in Kontakt mit dem Öffnungsrand aufgeschoben ist.

2. Behälter nach Anspruch 1, **dadurch gekennzeichnet, dass** die Verschlusssicherung einstückig aus Kunststoff geformt ist, so dass die Scharnierverbindung (8,9) ein Filmscharnier bildet.

3. Behälter nach Anspruch 2, **dadurch gekennzeichnet, dass** die Scharnierverbindung durch mindestens zwei biegsame, ihren Käfigring (6) mit ihrem Deckelteil verbindende Stege (8, 9) gebildet ist.

4. Behälter nach Anspruch 1, **dadurch gekennzeichnet, dass** die Rastmittel der Verschlussicherung aus mindest einem Rasteingriffsteil (10, 11) und mindestens einer zugehörigen Rastaufnahme (12, 13) bestehen, die einerseits am Käfigring (6) und anderseits am Deckelteil (7) angeformt sind.

5. Behälter nach Anspruch 4, **dadurch gekennzeichnet, dass** die mindestens eine Rastaufnahme (12, 13) beidseitig offen ist und einen sich in Rasteingriffsrichtung konisch verjüngende und in einer Rastschulter (15) endende Innenprofilierung aufweist.

6. Behälter nach Anspruch 5, **dadurch gekennzeichnet, dass** das mindestens eine Rasteingriffsteil (10, 11) als aussen am Deckelteil (7) angeformter, in eine Rastaufnahme (12,13) einfügbarer Zapfen (16) ausgeführt ist, der an einem Teil seines Umfanges einen eine Schulter bildenden, nasenförmigen Querschnitt aufweist, durch den eine Rastklinke (17) gebildet ist.

7. Behälter nach Anspruch 6, **dadurch gekennzeichnet, dass** der Zapfen (16) des Rasteingriffsteils (10,11) einen nach unten offenen Hohlkörper bildet.

## Claims

1. Container having an outwardly protruding opening edge, a snap-on lid (3) connected to this by its overlapping edge (4) in sealingly latched engagement, and having a sealing device, which non-detachably connects it to the container (1) without destruction, wherein, on one hand, the sealing device has a cage ring (6), such that it surrounds the container (1) below its opening edge, and, on the other hand, a lid part (7), which partially covers the snap-on lid (3) of the container (1), wherein the cage ring (6) is connected to the cover part (7) in a hinge-like manner and has latching means (10 - 13) in engagement without destruction in a non-detachable manner on at least one region opposite the hinge connection (8, 9) transverse to the container, said latching means latching the cage ring (6) and the cover part (7), snapped over this, of the sealing device to each other, such that they inherently enclose the snap-on lid (3), **characterised in that** the cage ring (6) is shifted from below onto the container (1) until it makes contact with the opening edge.

2. Container according to claim 1, **characterised in that** the sealing device is formed integrally from plastic, such that the hinge connection (8, 9) forms a film hinge.

3. Container according to claim 2, **characterised in that** the hinge connection is formed by at least two flexible webs (8, 9) connecting their cage ring (6) to their lid part.

4. Container according to claim 1, **characterised in that** the latching means of the sealing device consist of at least one latch engagement part (10, 11) and at least one corresponding latch receiver (12, 13), which, on one hand, are moulded on the cage ring (6) and, on the other hand, on the cover part (7).

5. Container according to claim 4, **characterised in that** the at least one latch receiver (12, 13) is open on both sides and has an inner profiling that tapers conically in the latch engagement direction and ends in a latching shoulder (15).

6. Container according to claim 5, **characterised in that** at least one latch engagement part (10, 11) is designed as a pin (16) that is externally moulded on the lid part (7) and that can be introduced into a latch receiver (12, 13), said pin having a nose-shaped cross-section forming a shoulder on one part of its periphery, a latching pawl (17) being formed by said cross-section.

7. Container according to claim 6, **characterised in that** the pin (16) of the latching engagement part (10, 11) forms a downwardly open hollow body.

## Revendications

1. Récipient comportant un bord d'ouverture ressortant vers l'extérieur, un couvercle enclipsable (3) qui, par son bord en surplomb (4), s'enclenche de façon étanche sur ledit bord d'ouverture, ainsi qu'un organe de fermeture de sécurité qui solidarise ledit couvercle audit récipient (1) et qui ne peut être séparé sans être endommagé, ledit organe de fermeture de sécurité présentant, d'une part, un anneau d'enfermement (6) qui entoure le récipient (1) sous son bord d'ouverture et, d'autre part, une partie couvercle (7) recouvrant partiellement le couvercle enclipsable (3) du récipient (1), ledit anneau d'enfermement (6) étant relié par une charnière à la partie couvercle (7) et présentant, sur au moins une zone opposée à la liaison formant charnière (8, 9), transversalement au récipient, des moyens d'accrochage (10 - 13) mutuellement enclenchés et qui ne peuvent être séparés sans être endommagés, lesquels assurent l'interverrouillage de l'anneau d'enfermement (6) et de la partie couvercle (7) de l'organe de fermeture de sécurité rabattue sur ce dernier pour ainsi enserrer le couvercle enclipsable (3) ; **caractérisé en ce que** l'anneau d'enfermement (6) est glissé autour du récipient (1) par le dessous, jusqu'à venir au contact de son bord d'ouverture.

2. Récipient selon la revendication 1, **caractérisé en ce que** l'organe de fermeture de sécurité est réalisé d'un seul tenant dans une matière plastique, la liaison formant charnière (8, 9) consistant en une charnière active.

3. Récipient selon la revendication 2, **caractérisé en ce que** la liaison formant charnière est formée par au moins deux traverses (8, 9) flexibles reliant leur anneau d'enfermement (6) à leur partie couvercle.

4. Récipient selon la revendication 1, **caractérisé en ce que** les moyens d'accrochage de l'organe de fermeture de sécurité se composent d'au moins une partie d'accrochage formant bouchon (10, 11) et au moins un logement d'accrochage (12, 13) correspondant, présents d'une part sur l'anneau d'enfermement (6) et d'autre part sur la partie couvercle (7).

5. Récipient selon la revendication 4, **caractérisé en ce que** l'au moins un logement d'accrochage (12, 13) est ouvert des deux côtés et présente un profil intérieur qui va en rétrécissant dans le sens de l'enclenchement d'accrochage et qui se termine par un épaulement d'accrochage (15).

6. Récipient selon la revendication 5, **caractérisé en ce que** l'au moins une partie d'accrochage formant bouchon (10, 11) est conçue sous la forme d'un téton (16) réalisé sur l'extérieur de la partie couvercle (7) et susceptible de s'insérer dans un logement d'accrochage (12, 13), lequel téton présente, sur une partie de sa circonférence, une section transversale à saillie formant un épaulement constituant un cliquet d'accrochage (17).

7. Récipient selon la revendication 6, **caractérisé en ce que** le téton (16) de la partie d'accrochage formant bouchon (10, 11) forme un corps creux ouvert vers le bas.
